(19) Europäisches Patentamt
European Patent Office
Office européen des brevets

(11) Veröffentlichungsnummer: **0 164 298**
**A2**

(12) # EUROPÄISCHE PATENTANMELDUNG

(21) Anmeldenummer: **85730038.8**

(22) Anmeldetag: **08.03.85**

(51) Int. Cl.⁴: **C 07 J 63/00**, A 61 K 31/57, C 07 J 71/00

(30) Priorität: **13.03.84 DE 3409554**

(43) Veröffentlichungstag der Anmeldung: **11.12.85 Patentblatt 85/50**

(84) Benannte Vertragsstaaten: **AT BE CH DE FR GB IT LI LU NL SE**

(71) Anmelder: **SCHERING AKTIENGESELLSCHAFT Berlin und Bergkamen, Müllerstrasse 170/178 Postfach 65 03 11, D-1000 Berlin 65 (DE)**

(72) Erfinder: **Annen, Klaus, Dr., Osthofstrasse 80, D-4400 Münster-Albachten (DE)**
Erfinder: **Laurent, Henry, Dr., Glambecker Weg 21, D-1000 Berlin 28 (DE)**
Erfinder: **Hofmeister, Helmut, Dr., Weislingenstrasse 4, D-1000 Berlin 28 (DE)**
Erfinder: **Wiechert, Rudolf, Prof. Dr., Petzower Strasse 8A, D-1000 Berlin 39 (DE)**

(54) **Neue 6alpha-Methyl-D-homo-kortikoide.**

(57) 6α-Methyl-D-homo-kortikoide der allgemeinen Formel (I):

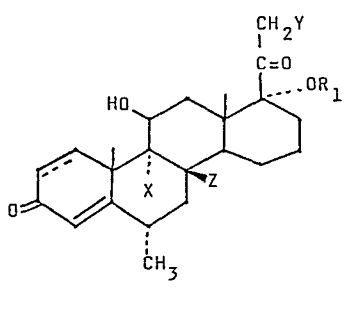

(I)

worin

die Bindung ..... eine Einfachbindung oder eine Doppelbindung darstellt und

X ein Wasserstoffatom, ein Fluoratom, ein Chloratom oder ein Bromatom,

Z ein Wasserstoffatom oder zusammen mit X eine Kohlenstoff-Kohlenstoff-Bindung,

$R_1$ eine Formylgruppe, eine 2 bis 8 Kohlenstoffatome enthaltende Alkanoylgruppe oder Alkoxyalkylgruppe, eine Benzoylgruppe oder eine Alkoxycarbonylgruppe mit 1 bis 6 Kohlenstoffatomen, und

Y ein Chloratom, eine Hydroxygruppe, eine Formylgruppe, eine Benzoyloxygruppe oder eine 2 bis 8 Kohlenstoffatome enthaltende Alkanoyloxygruppe, die gegebenenfalls durch eine 1 bis 3 Kohlenstoffatome enthaltende Alkoxygruppe substituiert sein kann, bedeuten, sind pharmakologisch wirksame Substanzen.

EP 0 164 298 A2

ACTORUM AG

Die Erfindung betrifft die im Patentanspruch 1 gekennzeichneten 6α-Methyl-D-homo-kortikoide, pharmazeutische Präparate, die diese Verbindungen enthalten, Verfahren zur Herstellung dieser Substanzen und letztlich Steroide, welche als Zwischenprodukte zur Synthese dieser D-Homo-Kortikoide verwendet werden.

Die neuen 6α-Methyl-D-homo-kortikoide können als Substituenten $R_1$ eine Formylgruppe, eine 2 bis 8 (vorzugsweise 2 bis 6) Kohlenstoffatome enthaltende Alkanoylgruppe oder Alkoxyalkylgruppe, eine Benzoylgruppe oder eine Alkoxycarbonylgruppe mit 1 bis 6 Kohlenstoffatomen enthalten. Geeignete Alkanoylgruppen $R_1$ sind beispielsweise die Acetylgruppe, Propionylgruppe, die Butyrylgruppe, die Isobutyrylgruppe, die Valerianylgruppe, die 3-Methylbutyrylgruppe, die Trimethylacetylgruppe oder die Hexanoylgruppen. Als Alkoxyalkylgruppen seien insbesondere Alkoxymethylgruppen, wie die Methoxymethylgruppe, die Ethoxymethylgruppe, die Propyloxymethylgruppe, die Isopropyloxymethylgruppe, die Butyloxymethylgruppe, die Isobutyloxymethylgruppe oder die tert.-Butyloxymethylgruppe beispielsmäßig genannt. Als Alkoxycarbonylgruppen seien die Methoxy-, die Ethoxy-, die Propyloxy-, die Isopropyloxy-, die Butyloxy-, die Isobutyloxy-, die tert.-Butyloxy- oder die Pentyloxy-carbonylgruppe beispielsmäßig benannt.

Als Substituenten Y können die 6α-Methyl-D-homo-kortikoide ein Chloratom, eine Hydroxygruppe, eine 2 bis 8 (vorzugsweise 2 bis 6) Kohlenstoffatome enthaltende Alkanoyloxygruppe oder eine Benzoyloxygruppe enthalten. Geeignete Alkanoyloxygruppen sind beispielsweise solche, die sich von den obengenannten Alkanoylgruppen ableiten. Diese Alkanoyloxygruppen können gegebenenfalls durch Alkoxygruppen substituiert sein; beispielsmäßig genannt seien die Methoxy-, die Ethoxy- und die Propyloxygruppe.

Die 6α-Methyl-D-homo-kortikoide der allgemeinen Formel I gemäß Patentanspruch 1, zeichnen sich bei topischer Applikation durch eine ausgeprägte antiinflammatorische Wirksamkeit aus. Darüberhinaus zeigen sie eine ausgezeichnete Dissoziation zwischen erwünschter topischer Wirksamkeit und unerwünschten systemischen Nebenwirkungen.

Es sei angemerkt, daß die 6α-Methyl-D-homo-kortikoide der allgemeinen Formel I gemäß Patentanspruch 1 mit X in der Bedeutung eines Wasserstoffatoms, eines Fluoratoms oder eines Chloratoms zur Anwendung in pharmazeutischen Präparaten geeigneter sind als solche, in denen der Substituent X ein Bromatom darstellt, da letztere in galenischen Zubereitungen weniger stabil sind. Andererseits sind aber die 9-Bromsteroide der allgemeinen Formel I gemäß Patentanspruch 1 wertvolle Zwischenprodukte zur Synthese der übrigen erfindungsgemäßen, pharmakologisch wirksamen 6α-Methyl-D-homo-kortikoide.

Die neuen 6α-Methyl-D-homo-kortikoide der allgemeinen Formel I gemäß Patentanspruch 1 eignen sich in Kombination mit den in der galenischen Pharmazie üblichen Trägermitteln zur lokalen Behandlung von Kontaktdermatitis, Ekzemen der verschiedensten Art, Neurodermatosen, Erythrodermie, Verbrennungen, Pruritis vulvae et ani, Rosacea, Erythematodes cutaneus, Psoriasis, Lichen ruber planus et verrucosus und ähnlichen Hauterkrankungen.

Die Herstellung der Arzneimittelspezialitäten erfolgt in üblicher Weise, indem man die Wirkstoffe mit geeigneten Zusätzen in die gewünschte Applikationsform, wie zum Beispiel: Lösungen, Lotionen, Salben, Cremes oder Pflaster, überführt. In den so formulierten Arzneimitteln ist die Wirkstoffkonzentration von der Applikationsform abhängig. Bei Lotionen und Salben wird vorzugsweise eine Wirkstoffkonzentration von 0,001 % bis 1 % verwendet.

Darüberhinaus sind die neuen Verbindungen gegebenenfalls in Kombination mit den üblichen Trägermitteln und Hilfsstoffen auch gut zur Herstellung von Inhalationsmitteln geeignet, welche zur Therapie allergischer Erkrankungen der Atemwege wie zum Beispiel des Bronchialasthmas oder der Rhinitis verwendet werden können.

Ferner eignen sich die neuen Kortikoide auch in Form von Kapseln, Tabletten oder Dragees, die vorzugsweise 10 bis 200 mg Wirkstoff enthalten und oral appliziert werden oder in Form von Suspensionen, die vorzugsweise 100 bis 500 mg Wirkstoff pro Dosiseinheit enthalten und rektal appliziert werden auch zur Behandlung allergischer Erkrankungen des Darmtraktes, wie der Kolitis ulcerosa und der Kolitis granulomatosa.

Die neuen 6α-Methyl-D-homo-kortikoide können nach dem in den Patentansprüchen 16 und 17 gekennzeichneten Verfahren hergestellt werden, welche unter den Bedingungen durchgeführt werden können, wie sie in den deutschen Patentanmeldungen Nr. P 26 45 104 und 26 45 107 sowie in der EP 34115 beschrieben sind.

Die nachfolgenden Ausführungsbeispiele dienen zur Erläuterung des erfindungsgemäßen Verfahrens.

Beispiel 1

a) Eine Suspension von 3.0 g Natriumacetat in 91 ml Chloroform und 91 ml Formaldehyddiethylacetal wird nach Zugabe von 3.0 g 21-Acetoxy-17aα-hydroxy-D-homo-4,9-pregnadien-3,20-dion mit 12.2 ml Phosphoroxychlorid versetzt und 0.5 h bei einer Badtemperatur von 65°C gerührt. Die abgekühlte Reaktionslösung wird mit soviel einer gesättigten Sodalösung behandelt, bis die wäßrige Phase alkalisch bleibt. Man trennt die organische Phase ab, wäscht neutral und engt nach dem Trocknen ein. Das Rohprodukt wird an 300 g Kieselgel mit einem Hexan-Essigester-Gradienten (0-50 % Essigester) gereinigt. Man isoliert 1.6 g 21-Acetoxy-17aα-hydroxy-6-methylen-D-homo-4,9-pregnadien-3,20-dion. Schmp. 165-167°C.

b) Eine Suspension von 150 mg Palladium/Aktivkohle in 8 ml Ethanol und 6 ml Cyclohexen wird 1 h bei 80°C Badtemperatur gerührt und nach Zugabe von 1.5 g 21-Acetoxy-17aα-hydroxy-6-methylen-D-homo-4,9-pregnadien-3,20-dion 12 h unter Rühren erhitzt. Nach dem Abkühlen wird der Katalysator abgesaugt, mit Methylenchlorid gewaschen und die vereinigten Filtrate mit 10 ml konz. Salzsäure 0.5 h bei Raumtemperatur gerührt. Man engt die Reaktionslösung auf 1/3 ihres Volumens ein, gibt auf Eiswasser und arbeitet wie üblich auf. Das Rohprodukt wird an 150 g Kieselgel mit einem Hexan-Essigester-Gradienten (0-50 % Essigester) gereinigt. Man erhält 1.2 g 21-Acetoxy-17aα-hydroxy-6α-methyl-D-homo-4,9-pregnadien-3,20-dion.

c) Eine Suspension von 1.1 g 21-Acetoxy-17aα-hydroxy-6α-methyl-D-homo-4,9-pregnadien-3,20-dion in 14 ml Propionsäure wird auf 0°C abgekühlt und nach dem Zutropfen von 5 ml Trifluoressigsäureanhydrid 2 h bei Raumtemperatur gerührt. Nach der Eiswasser-Kochsalz-Fällung filtriert man ab und arbeitet wie üblich auf. Das Rohprodukt wird an 100 g Kieselgel mit einem Hexan-Essigester-Gradienten (0-40 % Essigester) gereinigt. Ausbeute 820 mg 21-Acetoxy-6α-methyl-17aα-propionyloxy-D-homo-4,9-pregnadien-3,20-dion.

d) Eine Lösung von 0.5 g 21-Acetoxy-6α-methyl-17aα-propionyloxy-D-homo-4,9-pregnadien-3,20-dion in 8 ml Dioxan und 0.5 ml Wasser wird nach Zugabe von 380 mg N-Bromsuccinimid bei 20°C tropfenweise mit einer Lösung von 0.04 ml 70proz. Perchlorsäure in 0.6 ml Wasser versetzt. Man rührt 1 h bei 20°C Innentemperatur weiter, kühlt auf 15°C herunter und neutralisiert tropfenweise mit einer Lösung aus 1.6 g Natriumacetat und 1.0 g Natriumsulfit in 9.7 ml Wasser. Dabei darf die Innentemperatur 23°C nicht überschreiten. Nach Zugabe von 5 ml Methanol wird das Reaktionsgemisch 15 min bei Raumtemperatur und nach Zugabe von 20 ml Wasser 3 h bei 0°C weitergerührt. Schließlich saugt man den Niederschlag ab, wäscht den Rückstand mit Wasser und trocknet bei 70°C im Vakuumtrockenschrank. Das Rohprodukt wird aus Aceton/Hexan umkristallisiert. Man isoliert 360 mg 21-Acetoxy-9α-brom-11ß-hydroxy-6α-methyl-17aα-propionyloxy-D-homo-4-pregnen-3,20-dion.

e) 330 mg 21-Acetoxy-9α-brom-11ß-hydroxy-6α-methyl-17aα-propionyloxy-D-homo-4-pregnen-3,20-dion werden in 7 ml wasserfreiem Tetrahydrofuran gelöst und nach Zugabe von 1.1 ml Tributylzinnhydrid und 30 mg Azobisisobutyronitril 2 h refluxiert. Man engt im Vakuum ein und reinigt den Rückstand an 50 g Kieselgel mit einem Hexan-Essigester-Gradienten (0-50 % Essigester). Ausbeute 140 mg 21-Acetoxy-11ß-hydroxy-6α-methyl-17aα-propionyloxy-D-homo-4-pregnen-3,20-dion. Schmp. 136-137°C.

<u>Beispiel 2</u>

a) Eine Lösung von 9.5 g 21-Acetoxy-17aα-hydroxy-6α-methyl-D-homo-4,9-pregnadien-3,20-dion in 475 ml Dioxan wird mit 9.5 g Dichlorodicyanobenzochinon 15 h refluxiert. Nach dem Abkühlen und Abfiltrieren engt man das Filtrat zur Trockne ein. Das Rohprodukt wird an 750 g Kieselgel mit einem Hexan-Essigester-Gradienten (0-50 % Essigester) gereinigt.

Rohausbeute 5.73 g 21-Acetoxy-17aα-hydroxy-6α-methyl-D-homo-1,4,9-pregnatrien-3,20-dion, die in 20 ml siedendem Ethanol tropfenweise mit einer Lösung von 5.73 g $Na_2S_2O_5$ in 8 ml Wasser behandelt werden. Nach 2 h wird das Reaktionsgemisch in der Weise destilliert, daß das Volumen in der Destillationsblase durch Wasserzufuhr erhalten bleibt und das Brückenthermometer 99°C anzeigt. Man kühlt die Destillationsblase auf -20°C ab, filtriert ab, wäscht den Rückstand gründlich mit Wasser und löst ihn in Methylenchlorid. Die organische Lösung wird nach dem Trocknen über eine Kieselgelschicht filtriert und anschließend eingeengt. Ausbeute 4.7 g.

b) Eine Lösung von 1.0 g 21-Acetoxy-17aα-hydroxy-6α-methyl-D-homo-1,4,9-pregnatrien-3,20-dion in 13 ml Diethylenglykoldimethylether und 1.5 ml Propionsäureanhydrid wird mit 1.5 g N,N-Dimethylaminopyridin 31 h bei 80°C gerührt. Nach der Eiswasserfällung wird wie üblich aufgearbeitet und an 100 g Kieselgel mit einem Methylenchlorid/Aceton-Gradienten chromatographiert. Man isoliert 720 mg 21-Acetoxy-6α-methyl-17aα-propionyloxy-D-homo-1,4,9-pregnatrien-3,20-dion.

c) 0.5 g 21-Acetoxy-6α-methyl-17aα-propionyloxy-D-homo-1,4,9-pregnatrien-3,20-dion werden in 6 ml Dioxan gelöst und nach Zugabe von 0.5 g N-Bromsuccinimid tropfenweise mit 15 ml einer 10proz. Perchlorsäure versetzt. Man rührt 0.5 h bei Raumtemperatur, gibt auf Eiswasser und arbeitet wie üblich auf. Man isoliert 0.55 g 21-Acetoxy-9α-brom-11ß-hydroxy-6α-methyl-17aα-propionyloxy-D-homo-1,4-pregnadien-3,20-dion.

d) 0.5 g 21-Acetoxy-9α-brom-11ß-hydroxy-6α-methyl-17aα-propionyloxy-D-homo-1,4-pregnadien-3,20-dion werden analog Beispiel 1e) debromiert, aufgearbeitet und chromatographiert. Ausbeute 180 mg 21-Acetoxy-11ß-hydroxy-6α-methyl-17aα-propionyloxy-D-homo-1,4-pregnadien-3,20-dion.

Beispiel 3

a) Unter den Bedingungen des Beispiels 1d) werden 2.0 g 21-Acetoxy-17aα-hydroxy-6α-methyl-D-homo-4,9-pregnadien-3,20-dion mit N-Bromsuccinimid und Perchlorsäure umgesetzt, aufgearbeitet und gereinigt. Man erhält 1.6 g 21-Acetoxy-9α-brom-11ß,17aα-dihydroxy-6α-methyl-D-homo-4-pregnen-3,20-dion.

b) Analog Beispiel 1e) werden 1.5 g 21-Acetoxy-9α-brom-11ß,17aα-dihydroxy-6α-methyl-D-homo-4-pregnen-3,20-dion debromiert, aufgearbeitet und chromatographiert. Ausbeute 740 mg 21-Acetoxy-11ß,17aα-dihydroxy-6α-methyl-D-homo-4-pregnen-3,20-dion.

c) Eine Lösung von 700 mg 21-Acetoxy-11ß,17aα-dihydroxy-6α-methyl-D-homo-4-pregnen-3,20-dion in 8 ml methanolischer 0.2 N Kaliumhydroxidlösung wird 40 min bei 0°C gerührt. Man neutralisiert mit 10proz. Essigsäure und erhält nach der Eiswasserfällung und Aufarbeitung ein Rohprodukt, das an 50 g Kieselgel mit einem Methylenchlorid-Aceton-Gradienten gereinigt wird. Ausbeute 440 mg 11ß,17aα,21-Trihydroxy-6α-methyl-D-homo-4-pregnen-3,20-dion.

d) 420 mg 11ß,17aα,21-Trihydroxy-6α-methyl-D-homo-4-pregnen-3,20-dion und 42 mg Pyridiniumtoluolsulfonat werden in 20 ml Methyl-t-butylether (Driveron S) unter Argon suspendiert. Bei einer Innentemperatur von 58°C werden 5 ml Driveron S abdestilliert, der Ansatz auf 40°C abgekühlt, mit 1 ml Orthobuttersäuretriethylester versetzt und anschließend 2 h am Rückfluß gekocht, so daß keine ungelösten Bestandteile vorhanden waren. Man fügt 0.5 ml Pyridin hinzu und engt die Lösung zum 17aα,21-(1-Ethoxybutylidendioxy)-11ß-hydroxy-6α-methyl-D-homo-4-pregnen-3,20-dion als Öl ein.

e) Das rohe 17aα,21-(1-Ethoxybutylidendioxy)-11ß-hydroxy-6α-methyl-D-homo-4-pregnen-3,20-dion wird in 12 ml Methanol suspendiert und mit einem Gemisch aus 0.5 ml 0.1 M wäßriger Natriumacetat-Lösung und 4.3 ml 0.1 N wäßriger Essigsäure 1 h refluxiert. Man engt bis zur Trübung ein, gibt auf Wasser

und extrahiert mit Essigester. Die organische Phase wird mit Wasser gewaschen, über Natriumsulfat getrocknet und im Vakuum zur Trockne eingedampft. Das Rohprodukt wird an 50 g Kieselgel mit einem Hexan-Aceton-Gradienten gereinigt. Ausbeute 380 mg 17aα-Butyryloxy-11ß,21-dihydroxy-6α-methyl-D-homo-4-pregnen-3,20-dion.

Beispiel 4

a) Analog Beispiel 2b) werden 1.0 g 21-Acetoxy-17aα-hydroxy-6α-methyl-D-homo-1,4,9-pregnatrien-3,20-dion mit Benzoesäureanhydrid und N,N-Dimethylaminopyridin in Diethylenglykoldimethylether zu 0.7 g 21-Acetoxy-17aα-benzoyloxy-6α-methyl-D-homo-1,4,9-pregnatrien-3,20-dion umgesetzt, aufgearbeitet und gereinigt.

b) Unter den Bedingungen des Beispiels 2c) werden 0.7 g 21-Acetoxy-17aα-benzoyloxy-6α-methyl-D-homo-1,4,9-pregnatrien-3,20-dion mit N-Bromsuccinimid umgesetzt, aufgearbeitet und gereinigt. Ausbeute 680 mg 21-Acetoxy-17aα-benzoyloxy-9α-brom-11ß-hydroxy-6α-methyl-D-homo-1,4-pregnadien-3,20-dion.

c) 0.6 g 21-Acetoxy-17aα-benzoyloxy-9α-brom-11ß-hydroxy-6α-methyl-D-homo-1,4-pregnadien-3,20-dion werden analog Beispiel 1e) debromiert, aufgearbeitet und chromatographiert. Man isoliert 240 mg 21-Acetoxy-17aα-benzoyloxy-11ß-hydroxy-6α-methyl-1,4-pregnadien-3,20-dion.

Beispiel 5

a) Unter den Bedingungen des Beispiels 1c) wird 1.0 g 21-Acetoxy-17aα-hydroxy-6α-methyl-D-homo-1,4,9-pregnatrien-3,20-dion mit Eisessig und Trifluoressigsäureanhydrid zu 910 mg 17aα,21-Diacetoxy-6α-methyl-D-homo-1,4,9-pregnatrien-3,20-dion umgesetzt, aufgearbeitet und gereinigt.

b) Analog Beispiel 2c) werden 800 mg 17aα,21-Diacetoxy-6α-methyl-D-homo-1,4,9-pregnatrien-3,20-dion mit N-Bromsuccinimid und Perchlorsäure zu 830 mg 17aα,21-Diacetoxy-9α-brom-11ß-hydroxy-6α-methyl-D-homo-1,4-pregnadien-3,20-dion umgesetzt, aufgearbeitet und gereinigt.

c) Wie im Beispiel 1e) beschrieben, werden 750 mg 17aα,21-Diacetoxy-9α-brom-11ß-hydroxy-6α-methyl-D-homo-1,4-pregnadien-3,20-dion debromiert, aufgearbeitet und chromatographiert. Ausbeute 310 mg 17aα,21-Diacetoxy-11ß-hydroxy-6α-methyl-D-homo-1,4-pregnadien-3,20-dion.

Beispiel 6

a) 1.75 g 21-Acetoxy-17aα-hydroxy-6α-methyl-D-homo-1,4,9-pregnatrien-3,20-dion werden in 13 ml wasserfreiem Methylenchlorid und 8 ml Formaldehyddimethylacetal gelöst und portionsweise mit einem Gemisch aus 2.5 g Kieselgur W 20 und 1.25 g Phosphorpentoxid versetzt. Man rührt 45 min bei Raumtemperatur nach, saugt ab und eluiert den Rückstand mehrmals mit Methylenchlorid, das 3-5 % Triethylamin enthält. Das Rohprodukt wird an 250 g Kieselgel mit einem Methylenchlorid-Aceton-Gradienten gereinigt. Man isoliert 1.3 g 21-Acetoxy-17aα-methoxymethoxy-6α-methyl-D-homo-1,4,9-pregnatrien-3,20-dion.

b) 400 mg 21-Acetoxy-17aα-methoxymethoxy-6α-methyl-D-homo-1,4,9-pregnatrien-3,20-dion werden in 4.0 ml Dioxan mit 330 mg N-Chlorsuccinimid versetzt und nach tropfenweiser Zugabe von 2.0 ml einer 10proz. Perchlorsäurelösung 3 h bei Raumtemperatur gerührt. Man gibt auf eine Eiswasser-Kochsalz-Lösung und arbeitet wie üblich auf. Ausbeute 320 mg 21-Acetoxy-9α-chlor-11ß-hydroxy-17aα-methoxymethoxy-6α-methyl-D-homo-1,4-pregnadien-3,20-dion.

Beispiel 7

a) Unter den Bedingungen des Beispiels 3c) werden 2.0 g 21-Acetoxy-17aα-hydroxy-6α-methyl-D-homo-1,4,9-pregnatrien-3,20-dion zu 1.3 g 17aα,21-Dihydroxy-6α-methyl-D-homo-1,4,9-pregnatrien-3,20-dion verseift, aufgearbeitet und gereinigt.

b) 900 mg 17aα,21-Dihydroxy-6α-methyl-D-homo-1,4,9-pregnatrien-3,20-dion werden unter den Bedingungen des Beispiels 3d) zu 17aα,21-(1-Ethoxypropylidendioxy)-6α-methyl-D-homo-1,4,9-pregnatrien-3,20-dion umgesetzt und aufgearbeitet.

c) Das rohe 17aα,21-(1-Ethoxypropylidendioxy)-6α-methyl-D-homo-1,4,9-pregnatrien-3,20-dion wird analog Beispiel 3e) zu 810 mg 21-Hydroxy-6α-methyl-17aα-propionyloxy-D-homo-1,4,9-pregnatrien-3,20-dion hydrolysiert, aufgearbeitet und gereinigt.

d) 800 mg 21-Hydroxy-6α-methyl-17aα-propionyloxy-D-homo-1,4,9-pregnatrien-3,20-dion werden in 8 ml Pyridin und 4 ml Propionsäureanhydrid 1 h bei Raumtemperatur gerührt. Nach Eiswasserfällung und üblicher Aufarbeitung isoliert man 830 mg 6α-Methyl-17aα,21-dipropionyloxy-D-homo-1,4,9-pregnatrien-3,20-dion.

e) Unter den Bedingungen des Beispiels 2c) werden 800 mg 6α-Methyl-17aα,21-dipropionyloxy-D-homo-1,4,9-pregnatrien-3,20-dion mit N-Bromsuccinimid und Perchlorsäure umgesetzt, aufgearbeitet und gereinigt. Ausbeute 860 mg 9α-Brom-11ß-hydroxy-6α-methyl-17aα,21-dipropionyloxy-D-homo-1,4-pregnadien-3,20-dion.

f) Eine Lösung von 800 mg 9α-Brom-11ß-hydroxy-6α-methyl-17aα,21-dipropionyloxy-D-homo-1,4-pregnadien-3,20-dion in 11 ml Aceton wird nach Zugabe von 1.2 g Kaliumcarbonat 24 h bei Raumtemperatur gerührt. Man filtriert das Kaliumcarbonat ab und engt das Filtrat im Vakuum ein. Der Rückstand

wird an 50 g Kieselgel mit einem Hexan-Essigester-Gradienten gereinigt. Man isoliert 470 mg 9,11ß-Epoxy-6α-methyl-17aα,21-dipropionyloxy-D-homo-1,4-pregnadien-3,20-dion.

g) Eine auf -40°C abgekühlte Lösung von 1.7 ml $(HF)_n$/Pyridin wird nach Zugabe von 450 mg 9,11ß-Epoxy-6α-methyl-17aα,21-dipropionyloxy-D-homo-1,4-pregnadien-3,20-dion 5.5 h bei einer Temperatur zwischen -20°C und -10°C gerührt. Man gibt auf eine ammoniakalische Eiswasserlösung, filtriert den Niederschlag ab und arbeitet wie üblich auf. Das Rohprodukt wird an 50 g Kieselgel mit einem Methylenchlorid-Aceton-Gradienten gereinigt. Ausbeute 280 mg 9α-Fluor-11ß-hydroxy-6α-methyl-17aα,21-dipropionyloxy-D-homo-1,4-pregnadien-3,20-dion.

Beispiel 8

Eine Lösung von 450 mg 9,11ß-Epoxy-6α-methyl-17aα,21-dipropionyloxy-D-homo-1,4-pregnadien-3,20-dion in 45 ml Chloroform wird mit 9 ml einer 0.5 N Salzsäurelösung 2.5 h bei Raumtemperatur gerührt. Man verdünnt mit Methylenchlorid, wäscht mit einer Bikarbonatlösung und anschließend mit Wasser. Das Rohprodukt wird an 100 g Kieselgel mit einem Methylenchlorid-Aceton-Gradienten gereinigt. Ausbeute 240 mg 9α-Chlor-11ß-hydroxy-6α-methyl-17aα,21-dipropionyloxy-D-homo-1,4-pregnadien-3,20-dion.

Beispiel 9

Eine Lösung von 270 mg 17aα-Butyryloxy-11ß,21-dihydroxy-6α-methyl-D-homo-4-pregnen-3,20-dion in 2.8 ml Pyridin wird bei 0°C mit 0.12 ml Methoxyessigsäurechlorid tropfenweise versetzt und 1 h unter Eiskühlung gerührt. Nach der Eiswasser-Kochsalz-Fällung filtriert man ab und nimmt den Rückstand in Methylenchlorid auf. Die organische Lösung wird neutralgewaschen, über Natriumsulfat getrocknet und im Vakuum eingeengt. Das Rohprodukt

wird aus Aceton/Hexan kristallisiert. Man isoliert 140 mg 17aα-Butyryloxy-11ß-hydroxy-21-methoxyacetoxy-6α-methyl-D-homo-4-pregnen-3,20-dion.

Beispiel 10

a) 1.0 g 17aα,21-Dihydroxy-6α-methyl-D-homo-1,4,9-pregnatrien-3,20-dion wird unter den Bedingungen des Beispiels 3d) zu 17aα,21-(1-Methoxybutylidendioxy)-6α-methyl-D-homo-1,4,9-pregnatrien-3,20-dion umgesetzt und aufgearbeitet.

b) Das rohe 17aα,21-(1-Methoxybutylidendioxy)-6α-methyl-D-homo-1,4,9-pregnatrien-3,20-dion wird analog Beispiel 3e) zu 870 mg 17aα-Butyryloxy-21-hydroxy-6α-methyl-D-homo-1,4,9-pregnatrien-3,20-dion hydrolysiert, aufgearbeitet und gereinigt.

c) 790 mg 17aα-Butyryloxy-21-hydroxy-6α-methyl-D-homo-1,4,9-pregnatrien-3,20-dion werden in 7.9 ml Pyridin und 3.9 ml Acetanhydrid 1 h bei Raumtemperatur gerührt und wie üblich aufgearbeitet. Man erhält 810 mg 21-Acetoxy-17aα-butyryloxy-6α-methyl-D-homo-1,4,9-pregnatrien-3,20-dion.

d) Analog Beispiel 2c) werden 700 mg 21-Acetoxy-17aα-butyryloxy-6α-methyl-D-homo-1,4,9-pregnatrien-3,20-dion mit N-Bromsuccinimid und Perchlorsäure umgesetzt, aufgearbeitet und gereinigt. Ausbeute 720 mg 21-Acetoxy-9α-brom-17aα-butyryloxy-11ß-hydroxy-6α-methyl-D-homo-1,4-pregnadien-3,20-dion.

e) Unter den Bedingungen des Beispiels 1e) werden 700 mg 21-Acetoxy-9α-brom-17aα-butyryloxy-11ß-hydroxy-6α-methyl-D-homo-1,4-pregnadien-3,20-dion debromiert, aufgearbeitet und gereinigt. Man isoliert 310 mg 21-Acetoxy-17aα-butyryloxy-11ß-hydroxy-6α-methyl-D-homo-1,4-pregnadien-3,20-dion.

Beispiel 11

Eine Lösung von 300 mg 21-Acetoxy-9α-brom-11ß-hydroxy-6α-methyl-17aα-propionyloxy-D-homo-1,4-pregnadien-3,20-dion in 6 ml N-Methylpyrrolidon wird mit 300 mg Lithiumchlorid 1.5 h bei 80°C Badtemperatur gerührt. Nach der Eiswasser-Kochsalz-Fällung wird wie üblich aufgearbeitet und das Rohprodukt an 50 g Kieselgel mit einem Hexan-Essigester-Gradienten gereinigt. Ausbeute 130 mg 21-Acetoxy-11ß-hydroxy-6α-methyl-17aα-propionyloxy-D-homo-1,4,8-pregnatrien-3,20-dion.

Beispiel 12

a) Eine Lösung von 5.0 g 17aα,21-Dihydroxy-6α-methyl-D-homo-1,4,9-pregnatrien-3,20-dion in 50 ml Pyridin wird bei 0°C mit 3.8 ml Chlorameisensäureethylester 20 min gerührt. Nach der Eiswasser-Kochsalz-Fällung wird wie üblich aufgearbeitet. Man kristallisiert aus Aceton/Hexan um und isoliert 3.9 g 21-Ethoxycarbonyloxy-17aα-hydroxy-6α-methyl-D-homo-1,4,9-pregnatrien-3,20-dion.

b) Zu einer Suspension von 2.8 g Kupfer(I)-jodid in 55 ml wasserfreiem Tetrahydrofuran tropft man bei 0°C unter Argon 13.5 ml einer 1.6 M Lithiummethyl-Lösung. Man rührt 15 min bei 0°C weiter und kühlt die gelbliche Lösung auf -30°C ab. Nach tropfenweiser Zugabe einer Lösung von 2.2 g 21-Ethoxycarbonyloxy-17aα-hydroxy-6α-methyl-1,4,9-pregnatrien-3,20-dion in 55 ml wasserfreiem Tetrahydrofuran wird das Reaktionsgemisch 40 min bei -30°C nachgerührt, anschließend auf eine eiskalte gesättigte Ammoniumchlorid-Lösung gegeben und mit Essigester extrahiert. Die organischen Extrakte werden wie üblich aufgearbeitet und das Rohprodukt an 50 g Kieselgel mit einem Methylenchlorid-Aceton-Gradienten gereinigt. Ausbeute 1.3 g 17aα-Ethoxycarbonyloxy-21-hydroxy-6α-methyl-D-homo-1,4,9-pregnatrien-3,20-dion.

c) Analog Beispiel 10c) werden 1.2 g 17aα-Ethoxycarbonyloxy-21-hydroxy-6α-methyl-D-homo-1,4,9-pregnatrien-3,20-dion mit Acetanhydrid zu 1.1 g 21-Acetoxy-17aα-ethoxycarbonyloxy-6α-methyl-D-homo-1,4,9-pregnatrien-3,20-dion umgesetzt, aufgearbeitet und gereinigt.

d) Unter den Bedingungen des Beispiels 2c) werden 900 mg 21-Acetoxy-17aα-ethoxycarbonyloxy-6α-methyl-D-homo-1,4,9-pregnatrien-3.20-dion mit N-Bromsuccinimid und Perchlorsäure umgesetzt, aufgearbeitet und gereinigt. Man isoliert 890 mg 21-Acetoxy-9α-brom-17aα-ethoxycarbonyloxy-11ß-hydroxy-6α-methyl-D-homo-1,4-pregnadien-3,20-dion.

e) Analog Beispiel 1e) werden 800 mg 21-Acetoxy-9α-brom-17aα-ethoxycarbonyloxy-11ß-hydroxy-6α-methyl-D-homo-1,4-pregnadien-3,20-dion debromiert, aufgearbeitet und gereinigt. Ausbeute 370 mg 21-Acetoxy-17aα-ethoxycarbonyloxy-11ß-hydroxy-6α-methyl-D-homo-1,4-pregnadien-3,20-dion.

Beispiel 13

a) Eine Lösung von 1.0 g 17aα,21-(1-Ethoxypropylidendioxy)-6α-methyl-D-homo-1,4,9-pregnatrien-3,20-dion in 50 ml Dimethylformamid wird nach tropfenweiser Zugabe von 1 ml Trimethylchlorsilan 23 h bei 80°C Badtemperatur gerührt. Nach der Eiswasserfällung und üblichen Aufarbeitung reinigt man das Rohprodukt an 150 g Kieselgel mit einem Methylenchlorid-Aceton-Gradienten. Ausbeute 640 mg 21-Chlor-6α-methyl-17aα-propionyloxy-D-homo-1,4,9-pregnatrien-3,20-dion.

b) Analog Beispiel 2c) werden 600 mg 21-Chlor-6α-methyl-17aα-propionyloxy-D-homo-1,4,9-pregnatrien-3,20-dion mit N-Bromsuccinimid und Perchlorsäure umgesetzt, aufgearbeitet und gereinigt. Man isoliert 570 mg 9α-Brom-21-chlor-11ß-hydroxy-6α-methyl-17aα-propionyloxy-D-homo-1,4-pregnadien-3,20-dion.

c) Unter den Bedingungen des Beispiels 7f) werden 550 mg 9α-Brom-21-chlor-11ß-hydroxy-6α-methyl-17aα-propionyloxy-D-homo-1,4-pregnadien-3,20-dion zu 220 mg 21-Chlor-9,11ß-epoxy-6α-methyl-17aα-propionyloxy-D-homo-1,4-pregnadien-3,20-dion umgesetzt, aufgearbeitet und gereinigt.

d) Wie im Beispiel 7g) beschrieben, werden 200 mg 21-Chlor-9,11ß-epoxy-6α-methyl-17aα-propionyloxy-D-homo-1,4-pregnadien-3,20-dion mit $(HF)_n$/Pyridin zu 130 mg 21-Chlor-9α-fluor-11ß-hydroxy-6α-methyl-17aα-propionyloxy-D-homo-1,4-pregnadien-3,20-dion umgesetzt, aufgearbeitet und gereinigt.

Patentansprüche

1. 6α-Methyl-D-homo-kortikoide der allgemeinen Formel I

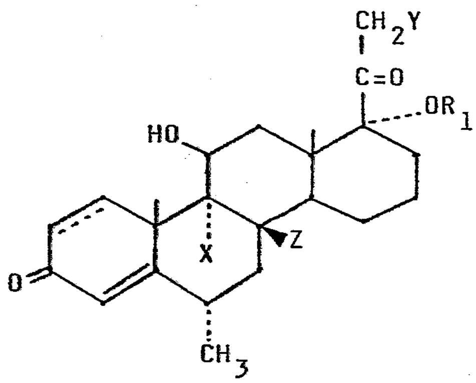

(I),

worin

die Bindung ..... eine Einfachbindung oder eine Doppelbindung darstellt und

X ein Wasserstoffatom, eine Fluoratom, ein Chloratom oder ein Bromatom,

Z ein Wasserstoffatom oder zusammen mit X eine Kohlenstoff-Kohlenstoff-Bindung,

$R_1$ eine Formylgruppe, eine 2 bis 8 Kohlenstoffatome enthaltende Alkanoylgruppe oder Alkoxyalkylgruppe, eine Benzoylgruppe oder eine Alkoxycarbonylgruppe mit 1 bis 6 Kohlenstoffatomen und

Y ein Chloratom, eine Hydroxygruppe, eine Formylgruppe, eine Benzoyloxygruppe oder eine 2 bis 8 Kohlenstoffatome enthaltende Alkanoyloxygruppe, die gegebenenfalls durch eine 1 bis 3 Kohlenstoffatome enthaltende Alkoxygruppe substituiert sein kann, bedeuten.

2. 21-Acetoxy-11β-hydroxy-6α-methyl-17aα-propionyloxy-D-homo-4-pregnen-3,20-dion.

3. 21-Acetoxy-11ß-hydroxy-6α-methyl-17aα-propionyloxy-D-homo-1,4-pregnadien-3,20-dion.

4. 17aα-Butyryloxy-11ß,21-dihydroxy-6α-methyl-D-homo-4-pregnen-3,20-dion.

5. 21-Acetoxy-17aα-benzoyloxy-11ß-hydroxy-6α-methyl-1,4-pregnadien-3,20-dion.

6. 17aα,21-Diacetoxy-11ß-hydroxy-6α-methyl-D-homo-1,4-pregnadien-3,20-dion.

7. 21-Acetoxy-9α-chlor-11ß-hydroxy-17aα-methoxymethoxy-6α-methyl-D-homo-1,4-pregnadien-3,20-dion.

8. 9α-Fluor-11ß-hydroxy-6α-methyl-17aα,21-dipropionyloxy-D-homo-1,4-pregnadien-3,20-dion.

9. 9α-Chlor-11ß-hydroxy-6α-methyl-17aα,21-dipropionyloxy-D-homo-1,4-pregnadien-3,20-dion.

10. 17aα-Butyryloxy-11ß-hydroxy-21-methoxyacetoxy-6α-methyl-D-homo-4-pregnen-3,20-dion.

11. 21-Acetoxy-17aα-butyryloxy-11ß-hydroxy-6α-methyl-D-homo-1,4-pregnadien-3,20-dion.

12. 21-Acetoxy-11ß-hydroxy-6α-methyl-17aα-propionyloxy-D-homo-1,4,8-pregnatrien-3,20-dion.

13. 21-Acetoxy-17aα-ethoxycarbonyloxy-11ß-hydroxy-6α-methyl-D-homo-1,4-pregnadien-3,20-dion.

14. 21-Chlor-9α-fluor-11β-hydroxy-6α-methyl-17aα-propionyloxy-D-homo-1,4-pregnadien-3,20-dion.

15. Pharmazeutische Präparate gekennzeichnet durch den Gehalt von ein oder zwei 6α-Methyl-D-homo-kortikoiden gemäß Patentanspruch 1 bis 14.

16. Verfahren zur Herstellung von 6α-Methyl-D-homo-kortikoiden der allgemeinen Formel I gemäß Patentanspruch 1, dadurch gekennzeichnet, daß man in an sich bekannter Weise

a) den Epoxidring eines Steroides der allgemeinen Formel II

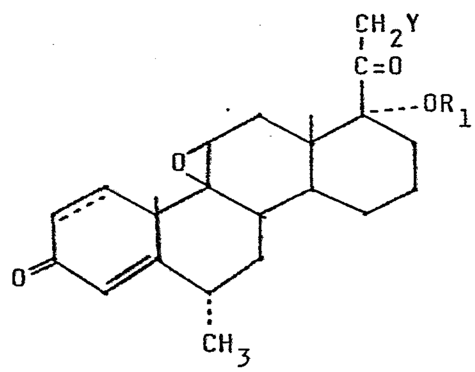

(II),

worin ....., $R_1$ und Y die im Patentanspruch 1 genannte Bedeutung besitzen, mit Fluorwasserstoff oder Chlorwasserstoff öffnet oder

b) an die $\Delta^{9(11)}$-Doppelbindung eines Steroids der allgemeinen Formel III

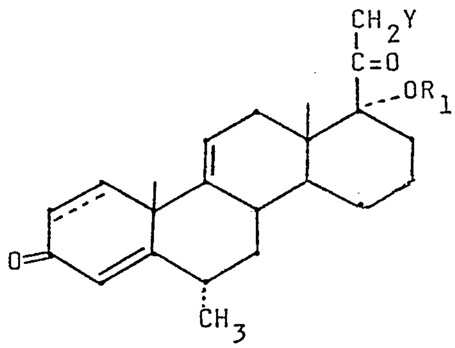

(III),

worin ....., $R_1$ und Y die im Patentanspruch 1 genannte Bedeutung besitzen unterchlorige oder unterbromige Säure anlagert und gewünschtenfalls aus den 9-Bromsteroiden der allgemeinen Formel I das in der 9-Position ständige Halogenatom reduktiv entfernt oder zur Einführung der $\Delta^8$-Doppelbindung Halogenwasserstoff eliminiert.

17. Verfahren zur Herstellung von 6α-Methyl-D-homo-kortikoiden der allgemeinen Formel I a

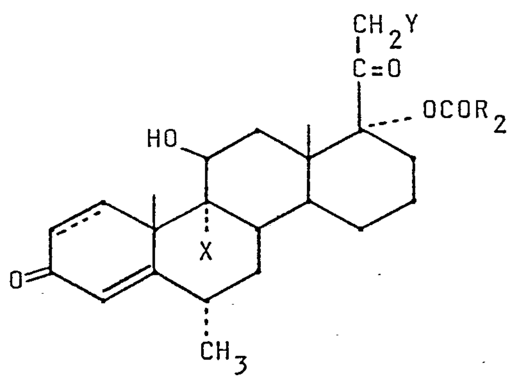

(Ia),

worin ....., X und Y die im Patentanspruch 1 genannte Bedeutung besitzen und

$R_2$ eine Alkylgruppe mit maximal 7 Kohlenstoffatomen, eine Alkoxygruppe mit maximal 6 Kohlenstoffatomen oder eine

Phenylgruppe darstellt, dadurch gekennzeichnet, daß man in an sich bekannter Weise

a) den Orthoester eines Steroides der allgemeinen Formel IV

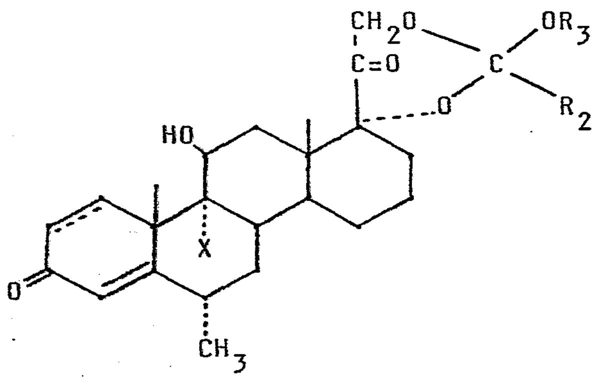

(IV),

worin ....., X und $R_2$ die obengenannte Bedeutung besitzen und

$R_3$ eine 1 bis 4 Kohlenstoffatome enthaltende Alkylgruppe darstellt,
mittels Säuren, Trimethylsilylchlorid oder Triphenylmethylchlorid spaltet, oder

b) eine 21-Acylgruppe eines Steroids der allgemeinen Formel V

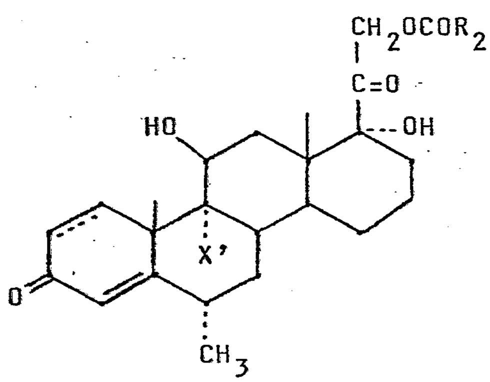

(V),

worin ..... und $R_2$ die obengenannte Bedeutung besitzen und X' ein Wasserstoff- oder Fluoratom bedeutet, umlagert und gegebenenfalls eine 21-ständige Hydroxylgruppe verestert oder gegen Chlor austauscht und gegebenenfalls ein in der 1,2-Position gesättigtes Steroid in dieser Position dehydriert.

18. Steroide der allgemeinen Formel VI

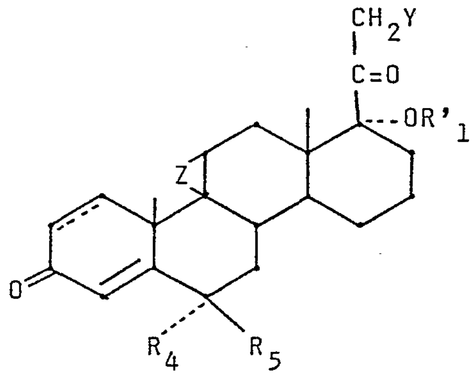

(VI),

worin ..... und Y die im Patentanspruch 1 genannte Bedeutung besitzen,

$R'_1$ für ein Wasserstoffatom oder $R_1$ mit der im Patentanspruch 1 genannten Bedeutung steht,

Z eine Kohlenstoff-Kohlenstoff-Bindung oder ein Sauerstoffatom bedeutet und worin

$R_4$ und $R_5$ gemeinsam eine Methylengruppe oder

$R_4$ ein Wasserstoffatom oder eine Methylgruppe und

$R_5$ ein Wasserstoffatom darstellen.

19. Steroide der allgemeinen Formel VII

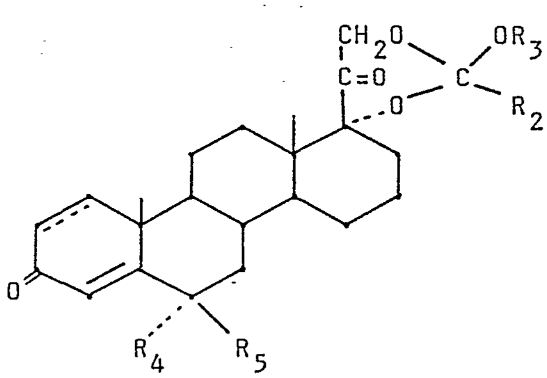

(VII),

worin ..... die im Patentanspruch 1, $R_2$ und $R_3$ die im Patentanspruch 17 sowie $R_4$ und $R_5$ die im Patentanspruch 18 genannte Bedeutung besitzen.

20. 21-Acetoxy-17aα-hydroxy-6-methylen-D-homo-4,9(11)-pregnadien-3,20-dion.

21. 21-Acetoxy-17aα-hydroxy-6α-methyl-D-homo-4,9,(11)-pregnadien-3,20-dion.

22. 21-Acetoxy-6α-methyl-17aα-propionyloxy-D-homo-4,9(11)-pregnadien-3,20-dion.

23. 21-Acetoxy-17aα-hydroxy-6α-methyl-D-homo-1,4,9(11)-pregnatrien-3,20-dion.

24. 21-Acetoxy-6α-methyl-17aα-propionyloxy-D-homo-1,4,9(11)-pregnatrien-3,20-dion.

25. 21-Acetoxy-17aα-benzoyloxy-6α-methyl-D-homo-1,4,9(11)-pregnatrien-3,20-dion.

26. 17aα,21-Diacetoxy-6α-methyl-D-homo-1,4,9(11)-pregnatrien-3,20-dion.

27. 21-Acetoxy-17aα-methoxymethoxy-6α-methyl-D-homo-1,4,9(11)-pregnatrien-3,20-dion.

28. 17aα,21-Dihydroxy-6α-methyl-D-homo-1,4,9(11)-pregnatrien-3,20-dion.

29. 17aα,21-(1-Ethoxypropylidendioxy)-6α-methyl-D-homo-1,4,9(11)-pregnatrien-3,20-dion.

30. 21-Hydroxy-6α-methyl-17aα-propionyloxy-D-homo-1,4,9-pregnatrien-3,20-dion.

31. 6α-Methyl-17aα,21-dipropionyloxy-D-homo-1,4,9(11)-pregnatrien-3,20-dion.

32. 9,11ß-Epoxy-6α-methyl-17aα,21-dipropionyloxy-D-homo-1,4-pregnadien-3,20-dion.

33. 17aα,21-(1-Methoxybutylidendioxy)-6α-methyl-D-homo-1,4,9(11)-pregnatrien-3,20-dion.

34. 17aα-Butyryloxy-21-hydroxy-6α-methyl-D-homo-1,4,9(11)-pregnatrien-3,20-dion.

35. 21-Acetoxy-17aα-butyryloxy-6α-methyl-D-homo-1,4,9(11)-pregnatrien-3,20-dion.

36. 21-Ethoxycarbonyloxy-17aα-hydroxy-6α-methyl-D-homo-1,4,9(11)-pregnatrien-3,20-dion.

37. 17aα-Ethoxycarbonyloxy-21-hydroxy-6α-methyl-D-homo-1,4,9(11)-pregnatrien-3,20-dion.

38. 21-Acetoxy-17aα-ethoxycarbonyloxy-6α-methyl-D-homo-1,4,9(11)-pregnatrien-3,20-dion.

39. 21-Chlor-6α-methyl-17aα-propionyloxy-D-homo-1,4,9(11)-pregnatrien-3,20-dion.

40. 21-Chlor-9,11ß-epoxy-6α-methyl-17aα-propionyloxy-D-homo-1,4-pregnadien-3,20-dion.